(19) Europäisches Patentamt
European Patent Office
Office européen des brevets

(11) EP 4 089 068 A1

(12) EUROPEAN PATENT APPLICATION
published in accordance with Art. 153(4) EPC

(43) Date of publication:
**16.11.2022 Bulletin 2022/46**

(21) Application number: **20908942.4**

(22) Date of filing: **16.07.2020**

(51) International Patent Classification (IPC):
***C07C 69/608*** *(2006.01)* ***C08K 5/12*** *(2006.01)*
***C08K 5/00*** *(2006.01)* ***C08L 27/06*** *(2006.01)*

(86) International application number:
**PCT/KR2020/009374**

(87) International publication number:
**WO 2021/137376 (08.07.2021 Gazette 2021/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.12.2019 KR 20190178656**

(71) Applicant: **Hanwha Solutions Corporation**
**Jung-gu**
**Seoul 04541 (KR)**

(72) Inventors:
- **KANG, Paul**
  **Daejeon 34128 (KR)**
- **KIM, Kidon**
  **Daejeon 34128 (KR)**
- **KIM, Minjeong**
  **Daejeon 34128 (KR)**
- **KIM, Yangjung**
  **Daejeon 34128 (KR)**

(74) Representative: **Herrmann, Uwe**
**Lorenz Seidler Gossel**
**Rechtsanwälte Patentanwälte**
**Partnerschaft mbB**
**Widenmayerstraße 23**
**80538 München (DE)**

# (54) ESTER-BASED COMPOUND AND USE THEREOF

(57) Provided are a novel ester-based compound and a use thereof. A use of a novel ester-based compound for improving heat resistance and compatibility as a plasticizer is provided. When the ester-based compound according to the present invention is used as the plasticizer of a heat-resistant resin composition, a synergistic effect on improvement of physical properties such as migration resistance and heating loss is provided to contribute to improvement of the physical properties of a molded article, and due to a rapid absorption rate and a short melting time, processability of the heat-resistant resin composition may be improved.

EP 4 089 068 A1

## Description

[Technical Field]

The present invention relates to a novel ester-based compound and a use thereof.

[Background Art]

Polymer resins used in everyday life have been variously applied and used in each field of living and home appliances, clothing, automobiles, construction materials, packaging materials, or the like, according to each of their characteristics. In general, polymer resins selected from polyethylene (PE), polypropylene (PP), polystyrene (PS), polyurethane (PU), polyvinyl chloride (PVC), and the like are used universally. In particular, polyvinyl chloride has universal utility, since it has hard and soft properties, may be advantageously applied to various molding methods, and has excellent price competitiveness, and thus, it is applied to various fields of application ranging from household goods to industrial materials.

Polyvinyl chloride is used by adding a plasticizer thereto for implementing various physical properties, rather than used as a resin alone. The plasticizer serves to impart flexibility to the resin to improve physical properties such as processability and moldability. However, as the industry develops, the role of the plasticizer has been diversified to strengthen the properties such as volatility resistance, migration resistance, aging resistance, cold resistance, oil resistance, water resistance, and heat resistance, which are required in the field of application.

An example of an ester-based compound universally used as the plasticizer may include di-(2-ethylhexyl) phthalate (DEHP), diisononyl phthalate (DINP), di-2-propylheptyl phthalate (DPHP), diisodecyl phthalate (DIDP), or the like. However, since these are phthalate plasticizers and have an environmental hormone problem, their use tends to be limited. In addition, dioctyl terephthalate (DOTP), which is a representative non-phthalate-based plasticizer, has limitations in improving the physical properties of heat stability such as migration and volatility (heating loss), and the like, of products.

Thus, there is an urgent demand for research to provide an ester-based compound which may solve the problems of a conventional ester-based compound used as a plasticizer and sufficiently improve the physical properties of a conventional product in terms of various physical properties such as processability with a resin, an absorption rate, a volatility loss, a migration loss, and heat stability.

### [Disclosure]

[Technical Problem]

An object of the present invention is to provide an ester-based compound with both heat resistance and compatibility being improved and a use thereof.

Specifically, an object of the present invention is to provide an ester-based compound having a use as a plasticizer with excellent heating loss and migration resistance, an ester-based plasticizer composition including the same, and a method of preparing the same.

Specifically, another object of the present invention is to provide a vinyl chloride-based resin composition including the ester-based plasticizer composition and a molded article manufactured therefrom.

[Technical Solution]

In one general aspect, a compound represented by the following Chemical Formula 1, that is, a novel ester-based compound is provided:

[Chemical Formula 1]

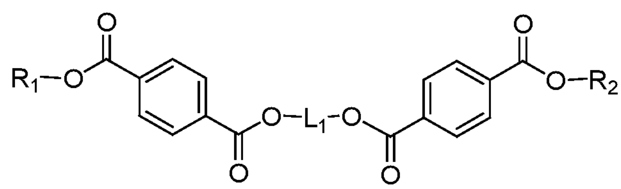

wherein

$R^1$ and $R^2$ are independently of each other C1-C10 alkyl; and
$L_1$ is C1-C10 alkylene, and non-adjacent $-CH_2-$ of the alkylene may be replaced in a manner in which -O- is not directly connected to each other.

The compound according to an exemplary embodiment of the present invention may be the compound of Chemical Formula 1 in which $R_1$ and $R_2$ are independently of each other C3-C10 alkyl, and L is C2-C6 alkylene.
The compound according to an exemplary embodiment of the present invention may be the compound of Chemical Formula 1 in which $R_1$ and $R_2$ are independently of each other C3-C10 alkyl, and L is selected from a structure represented by the following Chemical Formula A:

[Chemical Formula A]

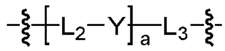

wherein

$L_2$ and $L_3$ are independently of each other ethylene or propylene;
Y is O; and
a is an integer of 1 or 2.

The compound according to an exemplary embodiment of the present invention may be the compound of Chemical Formula 1 in which $R_1$ and $R_2$ are independently of each other branched C3-C10 alkyl.
In another general aspect, an ester-based plasticizer composition includes the compound represented by Chemical Formula 1.
The ester-based plasticizer composition according to an exemplary embodiment of the present invention may include 30 wt% or more of the compound represented by Chemical Formula 1, based on a total weight.
In another general aspect, a method of preparing an ester-based plasticizer composition including the compound represented by Chemical Formula 1 includes: mixing a mixture of a polyhydric alcohol and a monohydric alcohol with a terephthalic acid and then performing an esterification reaction in the presence of a catalyst.
In the method of preparing an ester-based plasticizer composition according to an exemplary embodiment of the present invention, the polyhydric alcohol (A) and the monohydric alcohol (B) may be mixed at a weight ratio of 1:1 to 1:20 (A:B) in the mixture.
In the method of preparing an ester-based plasticizer composition according to an exemplary embodiment of the present invention, the ester-based plasticizer composition may include 30 wt% or more of the compound represented by Chemical Formula 1, based on a total weight.
In the method of preparing an ester-based plasticizer composition according to an exemplary embodiment of the present invention, the ester-based plasticizer composition may include a compound represented by the following Chemical Formula 2 as a remainder, based on the total weight:

[Chemical Formula 2]

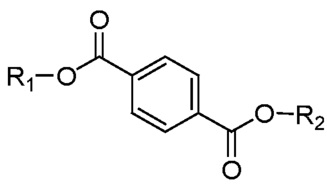

wherein
$R^1$ and $R^2$ are independently of each other C1-C10 alkyl.
In another general aspect, a vinyl chloride-based resin composition includes: the ester-based plasticizer composition described above.
The vinyl chloride-based resin composition according to an exemplary embodiment of the present invention may include 5 to 100 parts by weight of the ester-based plasticizer composition, based on 100 parts by weight of the vinyl chloride-based resin.

The vinyl chloride-based resin composition according to an exemplary embodiment of the present invention may further include one or more additives selected from a heat stabilizer, a filler, and the like.

The vinyl chloride-based resin composition according to an exemplary embodiment of the present invention may have a ratio of heating loss of 0.6% or less, the ratio of heating loss being measured with respect to the weight of an initial specimen measured at room temperature after being heated at 120°C for 72 hours.

Still another general aspect, a molded article manufactured from the vinyl chloride-based resin composition described above is provided.

[Advantageous Effects]

According to the present invention, an ester-based compound which is useful as a plasticizer for improving physical properties such as heat resistance and compatibility required for a heat-resistant resin composition such as a vinyl chloride-based resin is provided. In addition, the compound may improve processability which is the problem of a conventional ester-based compound universally used as a plasticizer.

According to the present invention, an ester-based plasticizer which is particularly excellent in properties such as migration resistance and heating loss may be provided in a very economical manner. In addition, according to the present invention, a manufacturing process is simplified and manufacturing costs may be reduced.

When the ester-based plasticizer according to the present invention is used as the plasticizer of a heat-resistant resin composition, a synergistic effect to improve physical properties such as migration resistance and heating loss is provided, thereby increasing compatibility, that is, plasticization efficiency and contributing to the improvement of the physical properties of a molded article. In addition, the ester-based plasticizer according to the present invention has a rapid absorption rate and a short melting time to improve the processability of a resin composition, and does not cause the physical properties of the heat-resistant resin to be deteriorated.

[Best Mode]

Hereinafter, the ester-based compound according to the present invention and the use thereof will be described in detail. Here, technical terms and scientific terms used in the present specification have the general meaning understood by those skilled in the art to which the present invention pertains unless otherwise defined, and description for the known function and configuration which may unnecessarily obscure the gist of the present invention will be omitted in the following description.

The singular form used in the present specification may be intended to also include a plural form, unless otherwise indicated in the context.

In addition, units used in the present specification without particular mention are based on weights, and as an example, a unit of % or ratio refers to a wt% or a weight ratio and wt% refers to wt% of any one component in a total composition, unless otherwise defined.

In addition, the numerical range used in the present specification includes all values within the range including the lower limit and the upper limit, increments logically derived in a form and span in a defined range, all double limited values, and all possible combinations of the upper limit and the lower limit in the numerical range defined in different forms. Unless otherwise defined in the specification of the present invention, values which may be outside a numerical range due to experimental error or rounding of a value are also included in the defined numerical range.

The term "comprise" in the present specification is an open-ended description having a meaning equivalent to the term such as "is/are provided", "contain", "have", or "is/are characterized", and does not exclude elements, materials, or processes which are not further listed.

The term "alkyl" in the present specification refers to a monovalent radical derived from an aliphatic hydrocarbon in the form of a linear or branched chain. In addition, "alkylene" refers to a divalent radical derived from an aliphatic hydrocarbon in the form of branched chain, ethylene may have a structural formula of $-CH_2CH_2-$, and propylene may have a structural formula of $-CH_2CH_2CH_2-$ or $-CH(CH_3)CH_2-$.

The term "compound according to the present invention" or "compound of the present invention" in the present specification refers to the compound represented by the following Chemical Formula 1, that is, an ester-based compound. In addition, the ester-based compound has a use as a plasticizer, and may have an equivalent meaning to an ester-based plasticizer.

The present inventors deepened the study of a plasticizer for improving heat resistance as well as processability with a heat-resistant resin such as a vinyl chloride-based resin, and invented a novel ester-based compound, that is, an ester-based plasticizer by a combination of alcohols having different structural characteristics from each other. In addition, it was confirmed that the ester-based compound according to the present invention may be provided in a very economical manner by adjusting the combination of alcohols having different structural characteristics from each other and their ratio at the same time, the objects described above may be achieved. In particular, it was confirmed that the ester-based

compound according to the present invention may implement a surprisingly improved synergistic effect on migration resistance, volatility resistance, and the like, and thus, the present invention is to be suggested.

The present invention is characterized by using an alcohol mixture having different structural characteristics, that is, a mixture of a monohydric alcohol and a polyhydric alcohol. In particular, the alcohol mixture according to the present invention may be a mixture in which the monohydric alcohol is mixed in excess of the polyhydric alcohol, and the mixture may satisfy a weight ratio of 1:1 to 1:20.

When the composition is satisfied, a problem which may arise by release of the plasticizer itself may be effectively prevented. In addition, the ester-based compound has a high molecular weight, but implements equivalent hardness properties, as compared with a universally used ester-based compound, and thus, provides an advantage which is good for compatibility, such as processability and workability, thereby having high plasticization efficiency. That is, according to the present invention, an ester-based compound which may satisfy both heat resistance and compatibility, and a use thereof may be provided. In addition, the present invention is noted in terms of securing a cost reduction technology for providing an ester-based compound allowing implementation of the physical properties described above.

Hereinafter, the present invention will be described in detail.

The ester-based compound according to an exemplary embodiment of the present invention may be represented by the following Chemical Formula 1:

[Chemical Formula 1]

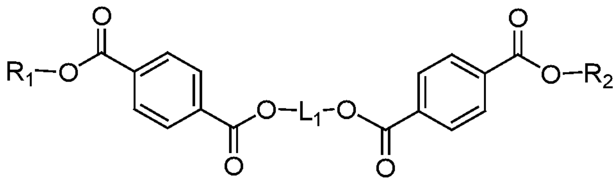

wherein

$R^1$ and $R^2$ are independently of each other C1-C10 alkyl; and
$L_1$ is C1-C10 alkylene, and non-adjacent $-CH_2-$ of the alkylene may be replaced in a manner in which O atoms are not directly connected to each other.

The non-adjacent $-CH_2-$ of the alkylene refers to alkylene except $-CH_2-$ adjacent to an oxygen atom connected to both ends of $L_1$ by a direct bond.

The ester compound represented by Chemical Formula 1 improves inferior quality of plasticization efficiency, migration, and the like of dioctyl terephthalate (DOTP) and the like which are a conventional ester-based compound universally used as a plasticizer, by the structural characteristics described above.

The ester-based compound may satisfy the following structures, in terms of showing a synergistic effect on the effect as such.

As an example, in Chemical Formula 1, $R_1$ and $R_2$ may be independently of each other C3-C10 alkyl, and L may be C2-C6 alkylene.

As an example, in Chemical Formula 1, $R_1$ and $R_2$ may be independently of each other C3-C10 alkyl, and L may be selected from the structure represented by the following Chemical Formula A:

[Chemical Formula A]

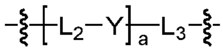

wherein

$L_2$ and $L_3$ are independently of each other ethylene or propylene;
Y is O; and
a is an integer of 1 or 2.

As an example, in Chemical Formula 1, $R_1$ and $R_2$ may be independently of each other branched C3-C10 alkyl.

As an example, in Chemical Formula 1, $R_1$ and $R_2$ may be independently of each other branched C5-C10 alkylene, and $L_1$ may be linear C2-C6 alkylene.

As an example, $R_1$ and $R_2$ may be independently of each other selected from methylbutyl, ethylbutyl, dimethylbutyl, methylpentyl, ethylpentyl, dimethylpentyl, methylhexyl, ethylhexyl, dimethylhextyl, and the like, and $L_1$ may be ethylene, propylene, butylene, pentylene, or hexylene, as linear alkylene.

As an example, $R_1$ and $R_2$ may be both 1-ethylhexyl or 2-ethylhexyl, and $L_1$ may be ethylene, propylene, butylene, pentylene, or hexylene, as linear alkylene.

As an example, in Chemical Formula 1, $R_1$ and $R_2$ may be independently of each other branched C5-C10 alkylene, and $L_1$ may be selected from the structure represented by Chemical Formula A. Here, in Chemical Formula A, $L_2$ and $L_3$ may be independently of each other ethylene or propylene, Y may be O, and a may be an integer of 1 or 2.

The ester-based compound according to an exemplary embodiment of the present invention is excellent in terms of properties such as migration resistance and heating loss, and also, improves the physical properties of a heat-resistant resin employing the ester-based compound, such as hardness, elongation, and tensile strength. In addition, the ester-based compound implements improved plasticization efficiency.

Specifically, the ester-based compound according to the present invention has a use as a plasticizer. That is, the ester-based compound according to the present invention may be an ester-based plasticizer.

The present invention provides an ester-based plasticizer composition including the ester-based compound according to the present invention and a method of preparing the same.

As an embodiment, the ester-based plasticizer composition according to an exemplary embodiment of the present invention may include 30 wt% or more of the ester-based compound represented by Chemical Formula 1, based on the total weight.

The ester-based plasticizer composition according to an exemplary embodiment of the present invention may include a compound represented by the following Chemical Formula 2 as a remainder, based on the total weight.

[Chemical Formula 2]

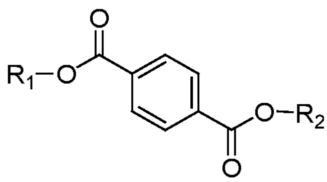

wherein

$R^1$ and $R^2$ are independently of each other C1-C10 alkyl.

The compound represented by Chemical Formula 2 may be a by-product produced when the compound represented by Chemical Formula 1 is prepared. Here, the compound represented by Chemical Formula 2 is combined with the ester-based compound according to the present invention and does not provide a disadvantage to plasticization efficiency, migration, and the like.

The ester-based plasticizer composition according to an exemplary embodiment provides an advantage of being excellent in heating loss according to a test method. Thus, the ester-based plasticizer according to the present invention is advantageous in providing excellent physical properties as a plasticizer and also solving the problems of a conventional plasticizer which currently causes environmental issues.

The ester-based plasticizer composition according to an exemplary embodiment of the present invention may be an ether-free plasticizer composition. Here, "ether-free" means that an ether compound is substantially not included or included at 1,000 ppm or less, 100 ppm or less, or 10 ppm or less in the ester-based plasticizer.

The ester-based plasticizer composition according to an exemplary embodiment of the present invention may be prepared by mixing a mixture of a polyhydric alcohol and a monohydric alcohol with a terephthalic acid and then performing an esterification reaction in the presence of a catalyst.

As described above, in the ester-based plasticizer composition according to the present invention, a combination of alcohols having different structural characteristics from each other is mixed at a predetermined ratio, and thus, the ester-based plasticizer prepared therefrom may implement a surprisingly improved synergistic effect on migration resistance and volatility resistance.

Specifically, the method of preparing an ester-based plasticizer composition according to an exemplary embodiment of the present invention is characterized by using a mixture of a polyhydric alcohol and a monohydric alcohol satisfying a weight ratio of 1:1 to 1:20.

When the alcohol mixture satisfying the weight ratio described above is used, the molecular weight of a plasticizer is improved, and the number of functional groups capable of interacting with a resin, that is, ester groups, is increased. In general, the aging resistant physical properties and the like of a plasticizer depend on a molecular weight, structural characteristics, and the like, and as the molecular weight of the plasticizer is higher, the aging resistant physical properties

are better, but compatibility with a resin tends to be reduced. However, according to the present invention, it is distinguished from conventional technologies in that both aging resistant physical properties and compatibility with a resin may be improved.

In addition, according to the present invention, an ester-based plasticizer composition of an embodiment in which at least two compounds are mixed is provided. This exerts an excellent synergistic effect on migration resistance and volatility resistance. Specifically, the ester-based plasticizer composition according to an exemplary embodiment of the present invention may be an embodiment in which at least one compound selected from the ester-based compound represented by Chemical Formula 1 and at least one compound selected from the compound represented by Chemical Formula 2 are mixed.

As an example, the ester-based plasticizer composition prepared by the preparation method according to the present invention may show a heating loss of 30% or less as compared with a dioctyl terephthalate (DOTP) single compound. Here, the heating loss may be a ratio of heating loss measured with respect to the weight of an initial specimen measured at room temperature (25°C) after being heated at 120°C for 72 hours.

As an example, the ester-based plasticizer composition prepared by the preparation method according to the present invention may show a migration amount of 1.5% or less as compared with a dioctyl terephthalate (DOTP) single compound. Here, the migration amount may be calculated by measuring the weight according to the following evaluation method and using the equation of (Wq2-Wq1)/Wi $\times$ 100.

In addition, when the alcohol mixture satisfying the weight ratio described above is used, an ester-based plasticizer composition having an improved heating loss and having excellent tensile strength, elongation, and heat resistant aging properties (e.g., tensile residual rate, stretch residual rate, and the like) may be provided. However, when an alcohol mixture having a weight ratio lower than the weight ratio described above is used, the content of the ester-based compound represented by Chemical Formula 1 is so low that the effect is not large, and when an alcohol mixture having a weight ratio higher than the weight ratio described above is used, molecular weight control is so poor that a high molecular weight ester-based plasticizer composition having high hardness is formed, and thus, it is difficult to maintain appropriate processability.

In the method of preparing an ester-based plasticizer composition according to an exemplary embodiment of the present invention, 2 to 18 parts by weight, more specifically 3 to 15 parts by weight of the primary alcohol may be included based on 1 part by weight of the polyhydric alcohol in the alcohol mixture.

In particular, when the weight ratio as such is satisfied, a plasticizer composition having a significantly reduced migration amount in the resin may be provided, which is thus preferred. In addition, the content of alcohol present as unreacted or an ether compound produced as a by-product is decreased to increase process efficiency. Meanwhile, when an adipic acid, an azelaic acid, or the like which is an aliphatic compound having two or more carboxylic group having structural characteristics similar to the terephthalic acid is used, the synergistic effect from the process conditions described above is not shown, which proves the selectivity of the preparation method according to the present invention.

Specifically, the step uses the alcohol mixture satisfying the weight ratio described above, and specifically, 10 to 50 wt% of the terephthalic acid, 10 to 70 wt% of the monohydric alcohol, and 1 to 25 wt% of the polyhydric alcohol are mixed, and then an esterification reaction is performed. More specifically, 20 to 45 wt% of the terephthalic acid, 20 to 60 wt% of the monohydric alcohol, and 3 to 20 wt% of the polyhydric alcohol, and most specifically, 30 to 40 wt% of the terephthalic acid, 30 to 60 wt% of the monohydric alcohol, and 4 to 16 wt% of the polyhydric alcohol are mixed, and then an esterification reaction is performed.

In the method of preparing an ester-based plasticizer composition according to an exemplary embodiment of the present invention, the monohydric alcohol may be an alcohol having 1 to 10 carbon atoms. In addition, when the monohydric alcohol is branched, an appropriate hardness is implemented and the mechanical properties of the resin may provide an advantage, which is thus preferred. A non-limiting example thereof may be selected from propanol, butanol, pentanol, hexanol, heptanol, octanol, and the like. Preferably, it may be 1-ethylhexyl, 2-ethylhexyl, or a combination thereof.

In the method of preparing an ester-based plasticizer composition according to an exemplary embodiment of the present invention, the polyhydric alcohol may be a compound including an alkylene group having 2 to 10 carbon atoms. A non-limiting example thereof may be selected from glycol-based compounds selected from glycerol, neopentyl glycol, ethylene glycol, diethylene glycol, triethylene glycol, propylene glycol, dipropylene glycol, and the like; and diol-based compounds selected from 1,2-propanediol, 1,3-propanediol, 1,4-butanediol, 1,3-butanediol, 2,3-butanediol, 1,3-pentanediol, 1,5-pentanediol, 1,6-hexanediol, 1,2-hexanediol, 3-methylenepentane-1,5-diol, 1,7-heptanediol, 1,8-octanediol, 1,9-nonanediol, 2-methyloctane-1,8-diol, and the like.

In the method of preparing an ester-based plasticizer composition according to an exemplary embodiment of the present invention, it is preferred that the polyhydric alcohol is linear in terms of excellent stability for tensile strength and elongation.

In the method of preparing an ester-based plasticizer composition according to an exemplary embodiment of the present invention, the most preferred ester-based plasticizer composition is derived from a combination of a terephthalic acid; a monohydric alcohol including 2-ethylhexanol; and a linear polyhydric alcohol.

In the method of preparing an ester-based plasticizer composition according to an exemplary embodiment of the present invention, the catalyst may be an organometallic catalyst including Sn-based or Ti-based catalyst, an acid catalyst including a sulfonic acid-based or sulfuric acid-based catalyst, or a mixed catalyst thereof. Here, the amount of the catalyst used is not limited, and may be used at a common amount of the catalyst used.

As an example, in the organometallic catalyst, the Ti-based organometallic catalyst may be selected from tetraalkyl titanate such as tetramethyl titanate, tetraethyl titanate, tetrapropyl titanate, tetraisopropyl titanate, tetrabutyl titanate, tetrampentyl titanate, tetrahexyl titanate, tetraoctyl titanate, tetranonyl titanate, tetradodecyl titanate, tetrahexadecyl titanate, tetraoctadecyl titanate, tetradecyl titanate, and tetraheptyl titanate; and tetraaryl titanate such as tetraphenyl titanate.

As an example, the acid catalyst may be selected from sulfuric acid, paratoluene sulfonic acid, methanesulfonic acid, ethanesulfonic acid, propanesulfonic acid, butanesulfonic acid, and the like.

As an example, the catalyst may be used at a common use amount, and specifically, may be used at 0.01 to 1 part by weight, based on a total of 100 parts by weight of the terephthalic acid, the monohydric alcohol, and the polyhydric alcohol, but is not limited thereto.

In the method of preparing an ester-based plasticizer composition according to an exemplary embodiment of the present invention, the step may be performed by batch injection of the terephthalic acid, the monohydric alcohol, and the polyhydric alcohol. In addition, continuous injection of continuously introducing the mixture of a monohydric alcohol and a polyhydric alcohol to the terephthalic acid or continuous injection of continuously introducing each of the monohydric alcohol and the polyhydric alcohol to the terephthalic acid may be performed, of course. In addition, the step may be performed by continuous injection of only the polyhydric alcohol.

In the method of preparing an ester-based plasticizer composition according to an exemplary embodiment of the present invention, the step may be performed by initiating the esterification reaction at a reaction temperature of 80°C or higher.

As an example, the reaction temperature of the step may be 135°C or higher.

As an example, the reaction temperature of the step may be 150°C or higher.

As an example, the reaction temperature of the step may be 170°C to 270°C.

As an example, the step may be performed in multiple steps at different reaction temperatures from each other. Specifically, the step may include a first step performed at a reaction temperature of 170 to 200°C; and a second step performed under a reaction temperature of 210 to 250°C.

In addition, the step may be performed at the reaction temperature described above for 10 minutes to 24 hours. The reaction time may be appropriately adjusted by an acid value obtained by the following Equation 1. A lower acid value is preferred, but the acid value is not limited as long as it is a value of 5 or less. Here, a high acid value means that an unreacted aromatic compound remains in the plasticizer, and may adversely affect the purity of the plasticizer.

[Equation 1]

$$\text{Acid value} = (\text{titration amount} \times 5.6 \times \text{factor}) / \text{sample amount}$$

wherein

when an alkaline solution used in titration is an aqueous 0.1 N KOH solution, the factor is 1.

In addition, the step may be performed under an inert atmosphere. The inert atmosphere refers to an inert gas atmosphere selected from nitrogen, argon, and the like.

The method of preparing an ester-based plasticizer composition according to an exemplary embodiment of the present invention may further include a purification step including a neutralization step after the step and a recovery step of an unreacted alcohol. The unreacted alcohol recovered by the purification as such may be reused, and thus, is advantageous in that it is continuously used in the reaction step to provide a more economical process.

The neutralization step may be performed using a common alkaline solution.

The step of recovering the unreacted alcohol may be a step of removing a reaction by-product as well as an alcohol present in an unreacted state, and may be a distillation step using a boiling point difference. When the distillation step is used, it is preferred that a difference in boiling points of the materials to be separated is 10°C or more. In addition, the distillation may be multistage distillation or mixed distillation. The multistage distillation may be separate distillation depending on the boiling point difference of each material to be separated, and the mixed distillation may be simultaneous distillation of the materials to be separated.

In addition, the present invention provides an ester-based plasticizer composition including the ester-based

compound prepared from the preparation method described above.

Specifically, the ester-based plasticizer composition according to the present invention may include a compound represented by the following Chemical Formulae 1 and 2. Specifically, the ester-based plasticizer composition may include 30 wt% or more of the ester-based compound represented by Chemical Formula 1.

[Chemical Formula 1]

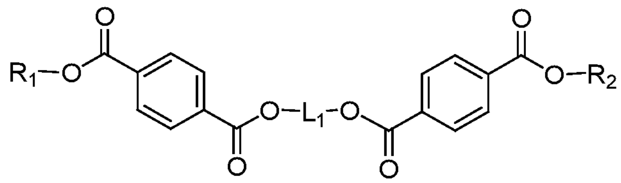

[Chemical Formula 2]

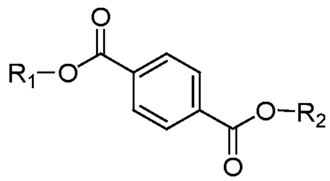

wherein

$R^1$ and $R^2$ are independently of each other C1-C10 alkyl; and
$L_1$ is C1-C10 alkylene, and non-adjacent $-CH_2-$ of the alkylene may be replaced in a manner in which O atoms are not directly connected to each other.

As an example, in Chemical Formulae 1 and 2, $R_1$ and $R_2$ may be independently of each other C3-C10 alkyl, and $L_1$ may be C2-C6 alkylene.

As an example, in Chemical Formulae 1 and 2, $R_1$ and $R_2$ may be independently of each other C3-C10 alkyl, and $L_1$ may be selected from the structure represented by the following Chemical Formula A:

[Chemical Formula A]

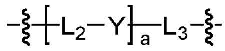

wherein

$L_2$ and $L_3$ are independently of each other ethylene or propylene;
Y is O; and
a is an integer of 1 or 2.

As an example, in Chemical Formulae 1 and 2, $R_1$ and $R_2$ may be independently of each other branched C3-C10 alkyl.

As an example, in Chemical Formulae 1 and 2, $R_1$ and $R_2$ may be independently of each other branched C5-C10 alkyl, and $L_1$ may be linear C2-C6 alkylene.

The ester-based plasticizer composition according to an exemplary embodiment of the present invention may be an ether-free plasticizer. Here, "ether-free" means that an ether compound is substantially not included or included at 1,000 ppm or less, 100 ppm or less, or 10 ppm or less in the ester-based plasticizer.

The ester-based plasticizer composition according to an exemplary embodiment of the present invention includes a conventional plasticizer, that is, a plasticizer such as dioctyl terephthalate (DOTP), but may overcome inferior quality of migration, volatility (heating loss), and the like which may be caused therefrom. In addition, an advantage of excellence in heating loss of a specimen prepared therefrom is provided. As a result as such, the ester-based plasticizer according to the present invention provides excellent physical properties as a plasticizer and also solves the problems

of a conventional plasticizer which currently causes environmental issues.

In addition, the ester-based plasticizer composition according to an exemplary embodiment of the present invention may solve not only the problem of the low-molecular weight plasticizer described above, but also the problem of conventional polymer plasticizers. Specifically, though the ether-based plasticizer composition according to the present invention has a molecular weight of 600 or more, it implements a hardness equivalent to the hardness of the low-molecular weight plasticizer described above. Thus, the ether-based plasticizer composition does not provide a disadvantage in processability with a heat-resistant resin such as a vinyl chloride-based resin, and implements high plasticization efficiency.

More specifically, according to the present invention, an ester-based plasticizer composition including 20 wt% or less of the compound represented by Chemical Formula 2 may be provided.

Most specifically, according to the present invention, an ester-based plasticizer composition including 18 wt% or less of the compound represented by Chemical Formula 2 may be provided.

The present invention provides a vinyl chloride-based resin composition including the ester-based plasticizer composition according to the present invention described above. Specifically, the vinyl chloride-based resin composition may include 5 to 100 parts by weight of the ester-based plasticizer composition, based on 100 parts by weight of the vinyl chloride-based resin.

The vinyl chloride-based resin composition according to an exemplary embodiment of the present invention may further include an additive selected from a heat stabilizer, a filler, and the like.

The heat stabilizer may be a composite heat stabilizer including two or more selected from Ca, Zn, Al, Mg, and the like.

As an example, 1 to 15 parts by weight of the heat stabilizer may be included, based on 100 parts by weight of the vinyl chloride-based resin. Specifically, the heat stabilizer may be included at 3 to 12 parts by weight, more specifically 5 to 10 parts by weight. When the heat stabilizer is used at the content described above, it helps to improve heat stability. In addition, since it has excellent compatibility and a synergistic effect with the ester-based plasticizer and the vinyl chloride-based resin according to the present invention, it shows better effect than other stabilizers. In addition, it may further include a non-metal stabilizer selected from benzophenol, triazole, acrylonitrile, and the like.

The filler may improve productivity and dry touch sensation of the vinyl chloride-based resin. The filler as such may be selected from calcium carbonate, clay, talc, diatomaceous earth, and the like.

As an example, 5 to 100 parts by weight of the filler may be included, based on 100 parts by weight of the vinyl chloride-based resin. In addition, according to the purpose, the filler may be used at a use amount of more than 100 parts by weight based on 100 parts by weight of the vinyl chloride-based resin, of course.

The vinyl chloride-based resin composition according to an exemplary embodiment of the present invention has an absorption rate and a short melting time for the vinyl chloride-based resin to increase the processability with the resin. In addition, the vinyl chloride-based resin composition according to the present invention may include a heat-resistant resin selected from an acryl resin, an ABS resin, a urethane resin, a polyester resin, and the like as well as the vinyl chloride-based resin, and also, may include various polymer resins.

The vinyl chloride-based resin composition according to the present invention implements particularly low heating loss and migration resistance, when manufactured into a wire covering material. In addition, the composition has a stretch residual rate and a tensile residual rate after excellent heating. In particular, the vinyl chloride-based resin composition according to the present invention may achieve a stretch residual rate after heating to an initial length of 80% or more, specifically 90% or more, and more specifically 95% or more, the elongation residual rate being calculated based on a length measured after heating at 120°C for 72 hours and the initial length measured at room temperature in accordance with the test method of ASTM D638. In addition, the composition may achieve a ratio of heating loss of 0.5% or less, specifically 0.45% or less, and more specifically even 0.2% or less, the ratio of heating loss being measured with respect to the initial weight measured at room temperature after being heated at 120°C for 72 hours.

In addition, the vinyl chloride-based resin composition according to the present invention may be applied to various embodiments of prescription according to the purpose. As an example, compound prescription, sheet prescription, plastisol prescription, and the like are included.

In addition, the present invention provides a molded article manufactured from the vinyl chloride-based resin composition described above. The molded article may be applied in various embodiments depending on the use and form according to the purpose, of course. Specifically, the molded article may be a wire coating material. In addition, it may be a flooring, an automotive interior material, a film, a sheet, a wallpaper, a tube, or the like.

Hereinafter, the present disclosure will be described in more detail by the following examples. However, the following examples are only a reference for describing the present invention in detail, and the present invention is not limited thereto and may be implemented in various forms. In addition, unless otherwise defined, all technical terms and scientific terms have the same meanings as those commonly understood by a person skilled in the art to which the present invention pertains. In addition, the terms used herein are only for effectively describing certain examples, and are not intended to limit the present invention.

In addition, unless otherwise stated in the present invention, the unit of temperature is all °C, and room temperature refers to 25°C.

(Evaluation method)

1) Hardness (ASTM D2240):

For each specimen, the needle of a hardness tester ("A" type) was completely lowered using a hardness scale (ASKER CL-150, unit: Shore A) in accordance with the method of ASTM D2240, and then a hardness value shown at room temperature after 10 seconds was read. The hardness was tested at 5 points for each specimen, and the average value was calculated.

2) Tensile strength, elongation (ASTM D638):

For each specimen, a cross head speed was pulled at 200 mm/min using U.T.M., which was a test instrument, and then a tensile strength and an elongation at the point where the specimen was cut were measured. The tensile strength ($kgf/cm^2$) was calculated by load value (kgf) / thickness (cm) × width (cm), and the elongation (%) was calculated by extension / initial strength × 100. The measurements were performed at room temperature.

In addition, the specimen was allowed to stand at 120°C for 72 hours using a gear oven, and the elongation and the tensile strength thereof were measured in the same manner. The results from the method of ASTM D638 at room temperature were divided by the results after heating to obtain a % values, which were shown as the tensile residual rate and the stretch residual rate.

3) Migration resistance:

Each specimen was cut into a circle having a diameter of 3 cm, and the initial weight (Wi) was measured to 4 decimal places. The specimen was inserted between two sheets of 3M oil paper (55 mm × 85 mm) of which the initial weight (Wq1) was measured and allowed to stand in an oven at 70°C for 4 days in a state of applying a load of 5 kg, and the migration amount was calculated by (Wq2-Wq1)/Wi × 100. At this time, the migration amount refers to a plasticizer outflow amount (%).

4) Heating loss:

For each specimen, the initial weight (Wi) was measured to 4 decimal places. The specimen was fixed in an oven at 120°C using a clamp, taken out after 72 hours, and stored in a thermostat (25°C) for 4 hours or more, the weight of the specimen (Wo) was measured, and the heating loss was calculated by (Wi-Wo)/Wi × 100.

(Example 1)

To a 1L reactor equipped with a temperature sensor, a mechanical agitator, a condenser, a decanter, and a nitrogen injector, 320 g of 2-ethylhexanol, 200 g of terephthalic acid, and 22.5 g of 1,4-butanediol were introduced, and the temperature was raised to 180°C with stirring under a nitrogen condition. After heating, 0.2 g of tetra-N-butyl titanate (TNBT) was introduced, the temperature was raised to 220°C, and an esterification reaction was performed for 8 hours. When the reaction was completed, cooling to 90°C was performed, a 1 M NaOH solution was introduced, and filtration was performed using diatomaceous earth. Thereafter, unreacted alcohol, water, and impurities were removed using a rotary evaporation concentrator, and an ester-based plasticizer composition as the final product was obtained.

The obtained ester plasticizer composition was analyzed by GPC to confirm a DOTP content (%) based on the total weight. The results are shown in the following Table 1.

In addition, the ester-based plasticizer composition prepared by the preparation method was used to manufacture a specimen. The manufacture of the specimen was performed by blending 400 g of PVC (polymerization degree: 1000) with 200 g of a plasticizer, 32 g of a composite heat stabilizer (RUP-110), and 80 g of calcium carbonate and working with a roll mill at 170°C for 3 minutes to manufacture a 1 mm sheet. Thereafter, press working was performed by preheating at 180°C for 3 minutes, heating for 10 minutes, and cooling for 3 minutes to manufacture a specimen having a thickness of 3 mm.

The specimen was used, and the results of the physical properties measured by the evaluation method are shown in Tables 1 and 2.

(Examples 2 to 12)

Ester plasticizers which were the final products were obtained in the same manner as in Example 1, except that the composition and the weight ratio of the alcohol mixture and the introduction amount and the introduction method of the polyhydric alcohol were changed as in the following Table 1.
The DOTP contents (%) of the obtained ester plasticizers based on the total weight were confirmed by GPC analysis, and the results are shown in the following Table 1.
In addition, the specimen was manufactured in the same manner as in Example 1, and the results of the physical properties measured by the evaluation method are shown in the following Tables 1 and 2.

(Comparative Example 1)

Commercially available dioctyl terephthalate (DOTP, Hanwha Chemical Corporation) was used.
The specimen was manufactured in the same manner as in Example 1, and the results of the physical properties measured by the evaluation method are shown in the following Tables 1 and 2.

(Table 1)

| | a:b[1)] (weight ratio) | Introduction amount[2)] and introduction method[3)] | | DOTP | Hardness | Migration amount | Heating loss | Tensile strength | Elongation |
|---|---|---|---|---|---|---|---|---|---|
| | | | | Content (%) | | (%) | (specimen, %) | ($kg/cm^2$) | (%) |
| Example 1 | 1:14.5 | 0.3 | A | 69.4 | 86.4 | 0.32 | 0.6 | 180 | 510 |
| Example 2 | 1:8.7 | 0.5 | A | 67.4 | 86.2 | 0.27 | 0.48 | 183 | 491 |
| Example 3 | 1:8.7 | 0.5 | B | 66.23 | 86.4 | 0.29 | 0.53 | 181 | 487 |
| Example 4 | 1:4.3 | 1 | A | 16.9 | 90.8 | 0.04 | 0.2 | 182 | 457 |
| Example 5 | 1:1 | 1 | A | 10.1 | 92.1 | 0.01 | 0.13 | 174 | 447 |
| Example 6 | 1:12.5 | 0.3 | A | 64.62 | 85.9 | 0.21 | 0.58 | 190 | 515 |
| Example 7 | 1:7.5 | 0.5 | A | 68.29 | 86.2 | 0.16 | 0.34 | 188 | 465 |
| Example 8 | 1:3.8 | 1 | A | 10.8 | 91.1 | 0.01 | 0.19 | 173 | 448 |
| Example 9 | 1:3.8 | 1 | B | 17.1 | 88.8 | 0.02 | 0.28 | 179 | 451 |
| Example 10 | 1:6.6 | 0.5 | A | 28.24 | 87.1 | 0.1 | 0.43 | 179 | 479 |
| Example 11 | 1:3.3 | 1 | A | 17.13 | 90.9 | 0.01 | 0.18 | 178 | 472 |
| Example 12 | 1:1 | 1 | A | 6.11 | 95.2 | 0.01 | 0.09 | 170 | 455 |
| Comparative Example 1 | - | | | 100 | 85.6 | 0.7 | 0.94 | 185 | 511 |

a:b[1)]: weight ratio between polyhydric alcohol (a) and monohydric alcohol (b)
Examples 1 to 5 = 1,4-butanediol:2-ethylhexanol
Examples 6 to 8 = 1,5-pentanediol:2-ethylhexanol
Examples 10 to 12 = 1,6-hexanediol:2-ethylhexanol
Introduction amount[2)]: introduction amount of polyhydric alcohol, based on 1 mol of terephthalic acid
Introduction method[3)]: A is batch injection; B is continuous injection.

(Table 2)

| | Examples | | | | | | | | | | | | Comparative Example 1 |
|---|---|---|---|---|---|---|---|---|---|---|---|---|---|
| | 1 | 2 | 3 | 4 | 5 | 6 | 7 | 8 | 9 | 10 | 11 | 12 | |
| Tensile residual rate (%) | 91.6 | 93.2 | 93.1 | 98.2 | 98.2 | 91.7 | 94.1 | 98.3 | 98.6 | 96.1 | 97.6 | 98.8 | 87.7 |
| Stretch residual rate (%) | 94.2 | 95.7 | 96.1 | 98.9 | 99.1 | 94.8 | 96.6 | 99.7 | 99.7 | 98.6 | 99.1 | 99.8 | 92.3 |

As shown in Table 1, it is recognized that when the plasticizer composition including the ester-based compound according to the present invention was employed, an excellent effect on migration resistance and heating loss was shown. Specifically, according to the present invention, it was confirmed that a migration amount of 45.7% or less (Example 1) as compared with Comparative Example 1 was implemented, and a significantly low migration amount of 0.01% was implemented. In addition, it was confirmed that a heating loss of 61.7% or less (Example 6) as compared with Comparative Example 1 was implemented and a significantly low heating loss of 0.09% was implemented. In addition, according to the present invention, the tensile strength and the elongation which were equivalent to or higher than dioctyl terephthalate (DOTP) used in Comparative Example 1 were implemented, and thus, it is expected that the present invention may be usefully applied as a material to replace DOTP.

As shown in Table 2, when the plasticizer composition including the ester-based compound according to the present invention was employed, the tensile strength and the elongation which were equivalent to or higher than dioctyl terephthalate (DOTP) were able to be implemented, of course, and an advantage in the tensile residual rate and the stretch residual rate was also able to be provided.

Hereinabove, although the present invention has been described by specific matters, Examples, and Comparative Examples, they have been provided only for assisting in the entire understanding of the present invention. Therefore, the present invention is not limited to the above Examples. Various modifications and changes may be made by those skilled in the art to which the present invention pertains from this description.

Therefore, the spirit of the present invention should not be limited to the above-described exemplary embodiments, and the following claims as well as all modified equally or equivalently to the claims are intended to fall within the scope and spirit of the invention.

## Claims

1. A compound represented by the following Chemical Formula 1:

[Chemical Formula 1]

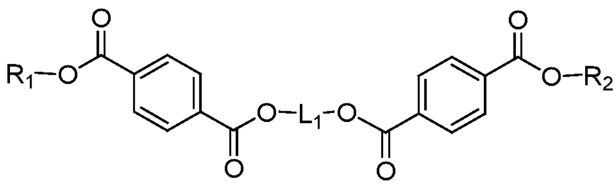

wherein

$R^1$ and $R^2$ are independently of each other C1-C10 alkyl; and
$L_1$ is C1-C10 alkylene, and non-adjacent $-CH_2-$ of the alkylene may be replaced in a manner in which O atoms are not directly connected to each other.

2. The compound of claim 1, wherein in Chemical Formula 1, $R_1$ and $R_2$ are independently of each other C3-C10 alkyl, and L is C2-C6 alkylene.

3. The compound of claim 1, wherein in Chemical Formula 1, $R_1$ and $R_2$ are independently of each other C3-C10 alkyl, and L is selected from a structure represented by the following Chemical Formula A:

[Chemical Formula A]

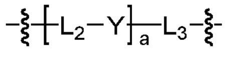

wherein

$L_2$ and $L_3$ are independently of each other ethylene or propylene;
Y is O; and
a is an integer of 1 or 2.

**4.** The compound of claim 1, wherein in Chemical Formula 1, $R_1$ and $R_2$ are independently of each other branched C3-C10 alkyl.

**5.** An ester-based plasticizer composition comprising a compound represented by the following Chemical Formula 1:

[Chemical Formula 1]

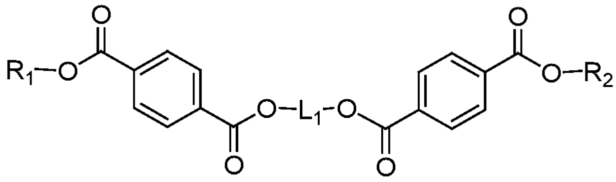

wherein

$R^1$ and $R^2$ are independently of each other C1-C10 alkyl; and
$L_1$ is C1-C10 alkylene, and non-adjacent $-CH_2-$ of the alkylene may be replaced in a manner in which O atoms are not directly connected to each other.

**6.** The ester-based plasticizer composition of claim 5, wherein 30 wt% or more of the compound represented by Chemical Formula 1 is comprised, based on a total weight of the ester-based plasticizer composition.

**7.** A method of preparing an ester-based plasticizer composition including a compound represented by the following Chemical Formula 1, the method comprising: mixing a mixture of a polyhydric alcohol and a monohydric alcohol with a terephthalic acid and then performing an esterification reaction in the presence of a catalyst:

[Chemical Formula 1]

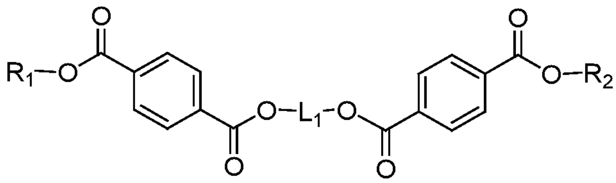

wherein

$R^1$ and $R^2$ are independently of each other C1-C10 alkyl; and
$L_1$ is C1-C10 alkylene, and non-adjacent $-CH_2-$ of the alkylene may be replaced in a manner in which O atoms are not directly connected to each other.

**8.** The method of preparing an ester-based plasticizer composition of claim 7, wherein the mixture of the polyhydric alcohol and the monohydric alcohol is mixed at a weight ratio of 1:1 to 1:20.

**9.** The method of preparing an ester-based plasticizer composition of claim 7, wherein 30 wt% or more of the compound represented by Chemical Formula 1 is included, based on a total weight of the ester-based plasticizer composition.

**10.** The method of preparing an ester-based plasticizer composition of claim 9, wherein a compound represented by the following Chemical Formula 2 is included as a remainder, based on the total weight of the ester-based plasticizer composition:

[Chemical Formula 2]

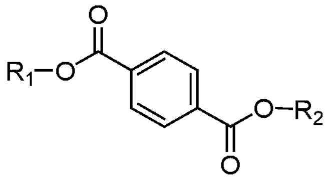

wherein
$R^1$ and $R^2$ are independently of each other C1-C10 alkyl.

**11.** A vinyl chloride-based resin composition comprising the ester-based plasticizer composition of claim 5 or 6.

**12.** The vinyl chloride-based resin composition of claim 11, wherein 5 to 100 parts by weight of the ester-based plasticizer composition is comprised, based on 100 parts by weight of the vinyl chloride-based resin.

**13.** The vinyl chloride-based resin composition of claim 11, further comprising: a heat stabilizer, a filler, or a combination thereof.

**14.** The vinyl chloride-based resin composition of claim 11, wherein the vinyl chloride-based resin composition has a ratio of heating loss of 0.6% or less, the ratio of heating loss being measured with respect to a weight of an initial specimen measured at room temperature after being heated at 120°C for 72 hours.

**15.** A molded article manufactured from the vinyl chloride-based resin composition of claim 11.

## INTERNATIONAL SEARCH REPORT

International application No.
**PCT/KR2020/009374**

**A. CLASSIFICATION OF SUBJECT MATTER**

**C07C 69/608**(2006.01)i; **C08K 5/12**(2006.01)i; **C08K 5/00**(2006.01)i; **C08L 27/06**(2006.01)i

According to International Patent Classification (IPC) or to both national classification and IPC

**B. FIELDS SEARCHED**

Minimum documentation searched (classification system followed by classification symbols)

C07C 69/608; C07C 67/03; C07C 67/08; C07C 67/54; C07C 69/44; C07C 69/74; C07C 69/82; C08G 63/18; C08G 63/181; C08K 5/12; C08K 5/00; C08L 27/06

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 테레프탈산(terephthalic acid), 에스테르(ester), 염화비닐수지(polyvinyl chloride resin), 가소제(plasticizer)

**C. DOCUMENTS CONSIDERED TO BE RELEVANT**

| Category* | Citation of document, with indication, where appropriate, of the relevant passages | Relevant to claim No. |
|---|---|---|
| X<br>Y | FIRDOVSI, S. T. et al. High molecular weight terephthalate esters (plasticizers) via transesterification reaction using MgO as heterogeneous catalyst under solvent-free condition. Neft Kimyasi va Neft E`mali Proseslari. 2005, vol. 2, pp. 82-91. See entire document. | 1-4,7-10<br>5-6,11-15 |
| X<br>Y | CN 102234227 A (FOSHAN YINMING TECHNOLOGY CO., LTD. et al.) 09 November 2011. See claims 6-8; and paragraphs [0018] and [0022]. | 1-2<br>5-6,11-15 |
| A | CN 104926648 A (SHANDONG YANHAI CONSTRUCTION RESOURCES CO., LTD.) 23 September 2015. See abstract; claims 1-4 and 10; and paragraphs [0011]-[0035], [0058]-[0059] and [0071]. | 1-15 |
| A | WO 2017-222232 A1 (LG CHEM, LTD.) 28 December 2017. See claims 1-8. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 December 2020** | **18 December 2020** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2020/009374

C. DOCUMENTS CONSIDERED TO BE RELEVANT

| Category* | Citation of document, with indication, where appropriate, of the relevant passages | Relevant to claim No. |
|---|---|---|
| A | KR 10-1973123 B1 (LG CHEM, LTD.) 29 April 2019. See claims 1, 4 and 7-17. | 1-15 |

Form PCT/ISA/210 (second sheet) (July 2019)

**INTERNATIONAL SEARCH REPORT**
**Information on patent family members**

International application No.
**PCT/KR2020/009374**

| Patent document cited in search report | Publication date (day/month/year) | Patent family member(s) | Publication date (day/month/year) |
|---|---|---|---|
| CN 102234227 A | 09 November 2011 | None | |
| CN 104926648 A | 23 September 2015 | None | |
| WO 2017-222232 A1 | 28 December 2017 | CN 108350154 A | 31 July 2018 |
| | | EP 3342794 A1 | 04 July 2018 |
| | | KR 10-2017-0142894 A | 28 December 2017 |
| | | KR 10-2135283 B1 | 17 July 2020 |
| | | TW 201808880 A | 16 March 2018 |
| | | US 2018-0298161 A1 | 18 October 2018 |
| KR 10-1973123 B1 | 29 April 2019 | CN 108779058 A | 09 November 2018 |
| | | EP 3293172 A2 | 14 March 2018 |
| | | TW 201806922 A | 01 March 2018 |
| | | US 10633511 B2 | 28 April 2020 |
| | | US 2018-0163019 A1 | 14 June 2018 |
| | | WO 2017-183874 A2 | 26 October 2017 |
| | | WO 2017-183874 A3 | 02 August 2018 |

Form PCT/ISA/210 (patent family annex) (July 2019)